# EUROPÄISCHE PATENTSCHRIFT

(11) **EP 2 408 732 B1**
(45) Veröffentlichungstag und Bekanntmachung des Hinweises auf die Patenterteilung: **23.01.2013**
(21) Anmeldenummer: 10709510.1
(22) Anmeldetag: 18.03.2010
(51) Int. Cl.: C07C 51/41, C07C 53/06, C07F 3/00

(54) **LÖSEMITTELFREIE HERSTELLUNG VON MAGNESIUMFORMIAT-BASIERTEM PORÖSEN METALLORGANISCHEN GERÜSTMATERIAL**
SOLVENT-FREE PRODUCTION OF MAGNESIUM FORMATE BASED POROUS METAL-ORGANIC FRAME MATERIAL
ÉLABORATION SANS SOLVANT DE MATÉRIAU SQUELETTE ORGANOMÉTALLIQUE POREUX À BASE DE FORMIATE DE MAGNÉSIUM

(30) Priorität: 20.03.2009 EP 09155685
(43) Veröffentlichungstag der Anmeldung: 25.01.2012
(73) Patentinhaber: BASF SE, 67056 Ludwigshafen (DE)
(72) Erfinder: LEUNG, Emi, 68161 Mannheim (DE); MUELLER, Ulrich, 67435 Neustadt (DE); COX, Gerhard, 67098 Bad Duerkheim (DE)
(86) Internationale Anmeldenummer: PCT/EP2010/053494
(87) Internationale Veröffentlichungsnummer: WO 2010/106121

(56) Entgegenhaltungen:
- WO-A1-2008/096985
- KR-A- 20050 052 929
- ROOD J A ET AL: "Synthesis, structural characterization, gas sorption and guest-exchange studies of the lightweight, porous metal-organic framework alpha-[Mg3(O2CH)6]" INORGANIC CHEMISTRY, AMERICAN CHEMICAL SOCIETY, Bd. 45, Nr. 14, 1. Januar 2006 (2006-01-01), Seiten 5521-5528, XP002539785 ISSN: 0020-1669 in der Anmeldung erwähnt
- DATABASE WPI Week 200617 Thomson Scientific, London, GB; AN 2006-164977 XP002583476 & WO 2006/018866 A1 (CIVIL CHEM ENG CO LTD) 23. Februar 2006 (2006-02-23)
- MALARD C ET AL: "Structure et stabilite thermique des deux formes du formiate de magnesium dihydrate" JOURNAL OF SOLID STATE CHEMISTRY, ORLANDO, FL, US LNKD- DOI:10.1016/0022-4596(82)90036-6, Bd. 41, Nr. 1, 1. Januar 1982 (1982-01-01) , Seiten 67-74, XP024194876 ISSN: 0022-4596 [gefunden am 1982-01-01]
- KENDALL, J. ET AL.: "Compound formation and solubility in systems of the type, formic acid : metal formate." J. AM. CHEM. SOC., Bd. 43, Nr. 7, 1921, Seiten 1470-1481, XP002583477
- GORSKI, A. ET AL.: "Origin of organic gaseous products formed in the thermal decomposition of formates" JOURNAL OF THERMAL ANALYSIS, Bd. 32, 1987, Seiten 1243-1251, XP002583478

## Beschreibung

Die vorliegende Erfindung betrifft ein Verfahren zur Herstellung eines Magnesiumformiat-basierten porösen metallorganischen Gerüstmaterials.

Magnesiumformiat als poröses metallorganisches Gerüstmaterial stellt ein interessantes metallorganisches Koordinationspolymer dar, welches aufgrund seiner Porosität für die Adsorption von Gasen geeignet ist.

Eine nähere Untersuchung dieses Stoffes wurde beispielsweise von J.A. Rood et al., Inorg. Chem. 45 (2006), 5521-5528 durchgeführt.

Ebenso wird in WO2009/115513 die Herstellung von Magnesiumformiat metallorganischem Gerüstmaterial sowie dessen Verwendung zur Methanspeicherung beschrieben.

In dem oben genannten Stand der Technik wird das Gerüstmaterial in N,N-Dimethylformamid als Lösemittel durchgeführt.

Trotz der guten Ergebnisse bei der Herstellung des Magnesiumformiat-basierten porösen metallorganischen Gerüstmaterials besteht ein Bedarf an weiteren Verfahren, die insbesondere den Einschluss von Lösemittel wie DMF vermeiden und in guten Ausbeuten und auf möglichst einfache Weise das gewünschte Gerüstmaterial ergeben.

Eine Aufgabe der vorliegenden Erfindung liegt somit darin, ein solches Verfahren zur Verfügung zu stellen.

Die Aufgabe wird gelöst durch ein Verfahren zur Herstellung eines Magnesiumformiat-basierten porösen metallorganischen Gerüstmaterials die Schritte enthaltend
(a) Zugeben von Magnesium oder Magnesiumoxid zu Ameisensäure;
(b) Rühren der Reaktionsmischung bei mindestens 75°C;
(c) Abfiltrieren der entstandenen Suspension.

Es hat sich nämlich gefunden, dass eine lösemittelfreie Synthese zu guten Resultaten führt, wobei die in flüssiger Form vorliegende Ameisensäure sowohl als Reagens als auch als Lösemittel fungiert. Solche Herstellverfahren werden typischerweise als "lösemittelfrei" bezeichnet, da keine nicht an der Reaktion beteiligte Flüssigkeit, welche üblicherweise in großem Überschuss im Vergleich zu den Reaktanten vorliegt, eingesetzt wird.

Der Begriff "Magnesiumformiat-basiert" soll zum Ausdruck bringen, dass das Gerüst des porösen metallorganischen Gerüstmaterials durch Formiat-Anionen und Magnesiumkationen aufgebaut ist. Dennoch kann ein Teil des Formiats auch in protonierter Form vorliegen, so dass das Gerüstmaterial auch "Fehlstellen" aufweisen kann. Darüber hinaus kann das metallorganische Gerüstmaterial aufgrund seiner Porosität in den Poren Essigsäure oder Formiat oder auch andere Stoffe enthalten, die jedoch nicht als Teil des Gerüstmaterials zu betrachten sind.

In Schritt (a) des erfindungsgemäßen Verfahrens wird Magnesium oder Magnesiumoxid zu Ameisensäure gegeben.

Es ist bevorzugt, dass die Zugabe unter einer Schutzgasatmosphäre wie einer Argonatmosphäre durchgeführt wird. Dies gilt insbesondere, wenn Magnesium eingesetzt wird. Sofern metallisches Magnesium eingesetzt wird, liegt dies vorzugsweise in Form von Magnesiumspänen vor. Die molare Menge an Ameisensäure im Verhältnis zu Magnesium oder Magnesiumoxid entspricht vorzugsweise mindestens einem 2,5-fachen molaren Überschuss. Weiterhin bevorzugt beträgt der Überschuss mindestens das 5-Fache.

Die Reinheit der Ameisensäure beträgt vorzugsweise mindestens 95%, weiter bevorzugt mindestens 98%, weiter bevorzugt mindestens 99%. Insbesondere wird reine Ameisensäure eingesetzt. Vorzugsweise ist die Ameisensäure wasserfrei.

Insbesondere beim Einsatz von Magnesiumoxid kann aufgrund der exothermen Reaktion eine Kühlung in Schritt (a) erforderlich sein. Die Zugabe erfolgt vorzugsweise dahingehend, dass die Temperatur weniger als 100°C, insbesondere von 50 bis 80°C beträgt.

Nach erfolgter Zugabe wird das gebildete Reaktionsgemisch in Schritt (b) des erfindungsgemäßen Verfahrens gerührt. Vorzugsweise erfolgt dies mindestens 30 Minuten lang, weiter bevorzugt mindestens 45 Minuten lang und insbesondere mindestens eine Stunde. Vorzugsweise erfolgt dies weniger als 10 Stunden, mehr bevorzugt weniger als 7,5 Stunden und insbesondere weniger als 5 Stunden.

Die Umsetzung kann unter Druck erfolgen, so dass höhere Temperaturen als die Siedetemperatur von Ameisensäure möglich sind. Vorzugsweise beträgt der Druck jedoch höchstens 2 bar (absolut). Weiter bevorzugt beträgt der Druck jedoch höchstens 1230 mbar (absoulut). Insbesondere bevorzugt findet die Umsetzung bei Atmosphärendruck statt. Hierbei kann es jedoch apparativ bedingt zu leichten Über- oder Unterdrücken kommen. Daher ist im Rahmen der vorliegenden Erfindung unter dem Begriff "Atmosphärendruck" derjenige Druckbereich zu verstehen, der sich aus dem tatsächlich vorliegenden Atmosphärendruck +/- 150 mbar ergibt.

Das Rühren in Schritt (b) wird mindestens bei einer Temperatur von 75°C durchgeführt. Vorzugsweise beträgt die Temperatur jedoch mindestens 90°C. Weiter bevorzugt beträgt die Temperatur jedoch höchstens 110°C, insbesondere ist ein Temperaturbereich von 95 bis 105°C bevorzugt. Ganz besonders bevorzugt erfolgt das Rühren in Schritt (b) des erfindungsgemäßen Verfahrens unter Rückfluss, insbesondere bei Atmosphärendruck.

Nach Schritt (b) des erfindungsgemäßen Verfahrens erfolgt in Schritt (c) des erfindungsgemäßen Verfahrens ein Filtrationsschritt. Aufgrund des entstandenen Magnesiumformiat-basierten porösen metallorganischen Gerüstmaterials ist eine Suspension entstanden, die entsprechend abfiltriert wird. Vorzugsweise erfolgt das Abfiltrieren in Gegenwart eines Lösemittels. Dieses Lösemittel kann vor dem Abfiltrieren, nach dem Abfiltrieren oder während des Abfiltrierens der Suspension zugefügt werden. Vorzugsweise wird die Suspension mit einem Lösemittel aufgenommen und das so entstandene Gemisch der Filtration zugeführt. Bei dem Lösemittel handelt es sich vorzugsweise um Aceton.

### Beispiele

### Beispiel 1 Herstellung des Magnesiumformiat-basierten metallorganischen Gerüstmaterials mit Hilfe von metallischem Magnesium

| | | | |
|---|---|---|---|
| 1) 5 g | Magnesium-Späne | (24,3 g/mol) | = 205,8 mmol |
| 2) 100 g | Ameisensäure | (46.0 g/mol) | = 2174 mmol |

a) Synthese: Ameisensäure wird unter Argon vorgelegt und Magnesium-Späne portionsweise innerhalb 1 h zugegeben (exotherm bis 40°C). Es wird 2h weitergerührt (Temperaturerhöhung auf 55°C, Lsg. wird trübe). Danach auf Rückfluß (RF)-Temperatur erhitzt und 1 h unter RF gekocht.
b) Aufarbeitung: Bei Raumtemperatur wird die entstandene Suspension in 250 ml Aceton eingerührt und abfiltriert sowie 2x mit je 100 ml Aceton nachgewaschen.
c) Trocknung: Das Gerüstmaterial wird in einer Porzellanschale 16h bei 130°C und 50 mbar im Vacuumtrockenschrank getrocknet.

| | |
|---|---|
| Farbe: | farblos |
| Ausbeute: | 21,3 g |
| Elementaranalyse | C: 20,8 Gew.%, H: 1,8 Gew.%, O: 56 Gew.%, Mg: 21,2 Gew.% |
| BET | 583 m²/g nach Langmuir |

### Bespiel 2 Herstellung des Magnesiumformiat-basierten metallorganischen Gerüstmaterials mit Hilfe von Magnesiumoxid

| | | | |
|---|---|---|---|
| 1) 16,6 g | Magnesiumoxid | (40,3 g/mol) | = 412 mmol |
| 2) 120 g | Ameisensäure | (46.0 g/mol) | = 2609 mmol |

a) Synthese: Ameisensäure wird in einem 0,25 I Vierhalskolben vorgelegt und Magnesiumoxid vorsichtig unter leichter Kühlung zugegeben (stark exotherm bis 75°C). Danach wird auf 100°C erhitzt und 1 h bei 100°C gerührt.
b) Aufarbeitung: Bei Raumtemperatur wird die entstandene dickflüssige Suspension in 300 ml Aceton eingerührt und abfiltriert sowie 2x mit je 200 ml Aceton nachgewaschen (gut filtrierbar).
c) Trocknung: In Porzellanschale dei das Gerüstmaterial 16h bei 130°C und 50 mbar im Vacuumtrockenschrank getrocknet.

| | |
|---|---|
| Farbe: | farblos |
| Ausbeute: | 42,3 g |
| Elementaranalyse | C: 20,9 wt.%, H: 1,8 wt.%, O: 56 wt.%, Mg: 21,0 wt.%, |
| BET | 556 m²/g nach Langmuir |

## Patentansprüche

1. Verfahren zur Herstellung eines Magnesiumformiat-basierten porösen metallorganischen Gerüstmaterials die Schritte enthaltend
(a) Zugeben von Magnesium oder Magnesiumoxid zu Ameisensäure;
(b) Rühren der Reaktionsmischung bei mindestens 75°C;
(c) Abfiltrieren der entstandenen Suspension.

2. Verfahren nach Anspruch 1, **dadurch gekennzeichnet, dass** bezogen auf das Magnesium Ameisensäure mit mindestens einem 2,5-fachen molaren Überschuss eingesetzt wird.

3. Verfahren nach Anspruch 1 oder 2, **dadurch gekennzeichnet, dass** Magnesium in Form von Magnesiumspänen eingesetzt wird.

4. Verfahren nach einem der Ansprüche 1 bis 3, **dadurch gekennzeichnet, dass** das Zugeben unter Schutzgasatmosphäre erfolgt.

5. Verfahren nach einem der Ansprüche 1 bis 4, **dadurch gekennzeichnet, dass** die Ameisensäure eine Reinheit von mindestens 95 % aufweist.

6. Verfahren nach einem der Ansprüche 1 bis 5, **dadurch gekennzeichnet, dass** das Rühren mindestens 30 Minuten erfolgt.

7. Verfahren nach einem der Ansprüche 1 bis 6, **dadurch gekennzeichnet, dass** das Rühren unter Atmosphärendruck erfolgt.

8. Verfahren nach einem der Ansprüche 1 bis 7, **dadurch gekennzeichnet, dass** das Rühren bei mindestens 90°C erfolgt.

9. Verfahren nach einem der Ansprüche 1 bis 8, **dadurch gekennzeichnet, dass** vor dem Abfiltrieren oder während des Abfiltrierens die entstandene Suspension mit einem Lösemittel in Kontakt gebracht wird.

10. Verfahren nach Anspruch 9, **dadurch gekennzeichnet, dass** das Lösemittel Aceton ist.

## Claims

1. A process for preparing a magnesium formate-based porous metal-organic framework, which comprises the steps
(a) addition of magnesium or magnesium oxide to formic acid;
(b)stirring of the reaction mixture at at least 75°C;
(c)isolation of the solid from the resulting suspension by filtration.

2. The process according to claim 1, wherein formic acid is used in an at least 2.5-fold molar excess based on the magnesium.

3. The process according to claim 1 or 2, wherein magnesium is used in the form of magnesium turnings.

4. The process according to any of claims 1 to 3, wherein the addition is carried out under a protective gas atmosphere.

5. The process according to any of claims 1 to 4, wherein the formic acid has a purity of at least 95%.

6. The process according to any of claims 1 to 5, wherein stirring is carried out for at least 30 minutes.

7. The process according to any of claims 1 to 6, wherein stirring is carried out under atmospheric pressure.

8. The process according to any of claims 1 to 7, wherein stirring is carried out at at least 90°C.

9. The process according to any of claims 1 to 8, wherein the suspension formed is brought into contact with a solvent before filtration or during filtration.

10. The process according to claim 9, wherein the solvent is acetone.
Solvent-free preparation of magnesium formate-based porous metal-organic framework

## Revendications

1. Procédé de fabrication d'un matériau de squelette métallo-organique poreux à base de formiate de magnésium, comprenant les étapes suivantes :
(a) l'ajout de magnésium ou d'oxyde de magnésium à de l'acide formique ;
(b) l'agitation du mélange réactionnel à au moins 75 °C ;
(c) la filtration de la suspension formée.

2. Procédé selon la revendication 1, **caractérisé en ce que** l'acide formique est utilisé en un excès molaire d'un facteur d'au moins 2,5 par rapport au magnésium.

3. Procédé selon la revendication 1 ou 2, **caractérisé en ce que** le magnésium est utilisé sous la forme de copeaux de magnésium.

4. Procédé selon l'une quelconque des revendications 1 à 3, **caractérisé en ce que** l'ajout a lieu sous une atmosphère de gaz protecteur.

5. Procédé selon l'une quelconque des revendications 1 à 4, **caractérisé en ce que** l'acide formique présente une pureté d'au moins 95 %.

6. Procédé selon l'une quelconque des revendications 1 à 5, **caractérisé en ce que** l'agitation a lieu pendant au moins 30 minutes.

7. Procédé selon l'une quelconque des revendications 1 à 6, **caractérisé en ce que** l'agitation a lieu à pression atmosphérique.

8. Procédé selon l'une quelconque des revendications 1 à 7, **caractérisé en ce que** l'agitation a lieu à au moins 90 °C.

9. Procédé selon l'une quelconque des revendications 1 à 8, **caractérisé en ce que** la suspension formée est mise en contact avec un solvant avant la filtration ou pendant la filtration.

10. Procédé selon la revendication 9, **caractérisé en ce que** le solvant est l'acétone.
